# EUROPEAN PATENT SPECIFICATION

(11) **EP 3 010 534 B1**
(45) Date of publication and mention of the grant of the patent: **30.05.2018**
(21) Application number: 14814542.8
(22) Date of filing: 17.06.2014
(51) Int. Cl.: A61K 39/00, A61K 38/28, A61K 31/00

(54) **PHARMACEUTICAL FORMULATIONS AND METHODS FOR ORAL DELIVERY OF BIOLOGICALLY ACTIVE INGREDIENT**
PHARMAZEUTISCHE FORMULIERUNGEN UND VERFAHREN ZUR ORALEN VERABREICHUNG BIOLOGISCHER WIRKSTOFFE
FORMULATIONS PHARMACEUTIQUES ET PROCÉDÉS D'ADMINISTRATION ORALE D'UN INGRÉDIENT BIOLOGIQUEMENT ACTIF

(30) Priority: 18.06.2013 IN 2648CH2013
(43) Date of publication of application: 27.04.2016
(73) Proprietor: Transgene Biotek Tld, Telangana State 502325 (IN)
(72) Inventor: KOLLIPARA, Koteswara Rao, Hyderabad Telangana State 500034 (IN); AHUJA, Rajiv, Hyderabad Telangana State 500049 (IN); PURANIK, Sarang S, Hyderabad Telangana State 500090 (IN); GREGORY, Russell-Jones, Middle Cove, New South Wales 2068 (AU)
(74) Representative: Boult Wade Tennant
(86) International application number: PCT/IB2014/062282
(87) International publication number: WO 2014/203156

(56) References cited:
- WO-A1-2004/045647
- WO-A2-2006/127361
- WO-A2-2013/021346
- JUN PAN: "Novel multi-biotin grafted poly(lactic acid) and its self-assembling nanoparticles capable of binding to streptavidin", INTERNATIONAL JOURNAL OF NANOMEDICINE, 1 January 2012 (2012-01-01), page 457, XP055329157, DOI: 10.2147/IJN.S24011
- SU YOUNG CHAE ET AL: "Preparation, Characterization, and Application of Biotinylated and Biotin-PEGylated Glucagon-Like Peptide-1 Analogues for Enhanced Oral Delivery", BIOCONJUGATE CHEMISTRY, vol. 19, no. 1, 1 January 2008 (2008-01-01), pages 334-341, XP055042315, ISSN: 1043-1802, DOI: 10.1021/bc700292v

## Description

### FIELD OF THE INVENTION

The present invention in general relates to pharmaceutical formulations and methods for oral delivery of biologically active ingredients to a vertebrate host via receptors present in the gastro intestinal tract. The invention also relates to processes for preparing suitable formulations for oral delivery of biologically active ingredients to a vertebrate host.

### BACKGROUND OF THE INVENTION

With the advancement of recombinant DNA technology and large scale production of recombinant proteins, there is an ever increasing list of bio-pharmaceutical products that are available for administration to patients. Unfortunately, the administration of these molecules must generally be via injections either once, twice or thrice weekly, depending upon the molecule and dose, or, in the case of insulin, up to four times daily. The administration of these molecules via the sub-cutaneous route has many disadvantages, including pain and swelling at the site of injection, highly variable intra and inter-subject variability in dosing, and large fluctuations in the serum profile of the sub-cutaneous administered drugs.

Administration of these proteins via alternative routes is precluded by the very low bioavailability of these molecules when given orally, transdermally, or via pulmonary administration. Despite this limitation, there are obvious benefits to oral delivery of the aforementioned molecules, including the ability to maintain a flatter serum profile of the administered protein, an increase in patient comfort and an increase in patient compliance. As such, the oral administration of proteins would be regarded as much more "patient friendly" and clearly more desirable than sub-cutaneous administration.

The oral route of administration may be a highly desirable however the gastro-intestinal tract poses a number of physical and chemical barriers to successful administration of therapeutic agents. The gastro intestinal tract is specifically designed to degrade proteins, and thus orally administered biologically active ingredients must withstand acidic milieu of the stomach as well as attack from endogenous proteolytic enzymes, without losing its biological activity. In addition to surviving the harsh milieu of the GI tract, the orally administered material must still have some means whereby it can cross the gastrointestinal barrier, enter into the systemic circulation and move to the site of activity. All of this must take place at an appropriate rate to ensure that correct biologically active dosage is delivered.

One of the previous approaches has been the use of intestinal transporters to enable the biologically active ingredient to cross the intestinal barrier and gain entry into the circulation. One such mechanism has previously been described by (Russell-Jones et al. 1995, 1996, Chalasani et al. 2007). These workers have utilized the uptake pathway for the water-soluble vitamin, vitamin B12 (cobalamin or cyanocobalamin). It has been shown that uptake of vitamin B12 is dependent upon binding of the vitamin by gastric intrinsic factor (IF), with subsequent recognition of the IF-VB12 complex by a membrane expressed receptor (IFCR) on the small intestinal epithelial cell (the enterocyte). Following binding of the IF-VB12 complex to the IFCR, the IF-VB12 is internalized, whereupon the IF is degraded and another VB12 binding protein, transcobalamin II binds to the VB12 and transports it across the cell (Russell-Jones et al. 2007). have shown that it is possible to link vitamin B12 to peptides, proteins and nanoparticles and use this normal, intrinsic factor-mediated transport system for vitamin B12, to co-transport the attached cargo from the intestine to the circulation following oral feeding. Whilst this transport system has been shown to have commercial utility in the oral delivery of peptides, proteins and nanoparticles, its utility is limited by a number of factors. Firstly, vitamin B 12 needs to be chemically modified to provide a suitable site for conjugation to the material to be limited, secondly the uptake capacity of the vitamin B12 transport system is rather low (1 nmole per feed), and thirdly vitamin B12 is relatively large molecule with a molecular weight of 1355.38, when compared with other potential carriers.

Biotin, a 244.31 molecular weight, water soluble vitamin, is an essential vitamin, which has been shown to have good bioavailability following oral feeding to humans. The average daily uptake of biotin is around 15-70 ug (60-300 nmole), which is considerably higher than that of vitamin B12 (1.34 ug, 1 nmole). Single feeding of 1 mg of biotin to human results in serum levels of 34,000 pmol/litre (8.3 ug/litre) 1-3 hours after feeding (Said et al. 2008).

One of the mechanisms for the uptake of biotin from the intestine is sodium dependent multivitamin transporter (SMVT), which has affinity for biotin, pantothenic acid and lipoate. This mechanism has been identified in rabbit small intestine, rat small intestine and colon and Caco-2 cells. A Na+ dependent transport of 3H-biotin was shown in Caco-2 transwell cultures (the SMVT) for biotin uptake in colonic epithelial cells (Said et al. 2008).

The possible use of the SMVT system described above, as an uptake mechanism for biotin-targeted peptides, proteins or nanoparticles in a similar fashion to that described for vitamin B12 conjugates, is precluded by the need to maintain a free carboxyl group on the biotin, pantothenic acid or lipoate molecules, which is lost during conjugation to the drug to be delivered orally. Furthermore, the SMVT utilizes a "pore-like" structure, which is only suitable for transport of very small molecules (less than 1,000 MW). Hence the SMVT has very limited utility for the transport of pharmaceuticals.

An alternative cellular uptake system for biotin, which is different from the SMVT has been described on several cancer cell lines. This system is capable of uptake of the free vitamin as well as biotin-polymer and biotin-nanoparticle conjugates. This uptake system is highly expressed on many aggressive tumour cell types and is separable from SMVT, as the later requires a free carboxyl group on the biotin (USP 2005/0220754 & USP 2006/0127310).

Whilst SMVT has been shown to be responsible for some uptake of biotin in the small intestine, the presence of an alternative cellular uptake system for biotin has, until recently, not been known.

In order to provide a solution to the prior art problems, the present invention discloses methods, complexes and formulations for using the transport system for the intestinal uptake and transport of biotin-conjugated biologically active ingredients.

### SUMMARY OF THE INVENTION

The following presents a simplified summary of the different aspects of the invention. The summary is not to be construed as an extensive overview of the disclosure and it does not identify key/critical elements or delineate the scope of the invention. The sole purpose of the below summary is to present few concepts of the invention in a simplified form as a prelude to the more detailed description that is presented in detailed description of the invention.

An objective of the present invention is to prepare pharmaceutical formulations and provide methods for oral delivery of biologically active ingredients to a vertebrate host via biotin specific receptors present in the gastro intestinal tract.

It is a preferred objective of the present invention to produce complexes of biotin or its analogues with the biologically active ingredient, without altering its therapeutic activity, to be delivered orally and utilizing the biotin transport system for effective oral delivery of such complexes.

Another preferred objective of the present invention is to use biotin, which is relatively smaller in size as compared to other essential vitamin like vitamin B12 and has a relatively high uptake capacity.

Another preferred objective of the present invention is to use biocytin, which is a natural derivative of biotin having a free carboxyl group and amino group for conjugation.

A further preferred objective of the present invention is to provide a new set of oral biologically active ingredient to which a biotin molecule, or analogue thereof, has been conjugated, thereby facilitating oral delivery of biologically active ingredient which does not have its own receptors in the gastrointestinal tract. See SU YOUNG CHAE ET AL: "Preparation, Characterization, and Application of Biotinylated and Biotin-PEGylated Glucagon-Like Peptide-1 Analogues for Enhanced Oral Delivery",BIOCONJUGATE CHEMISTRY, vol. 19, no. 1, 1 January 2008 (2008-01-01), pages 334-341, XP055042315,ISSN: 1043-1802, DOI: 10.1021/bc700292v as relevant prior art reference.

A further preferred objective of the present invention is to provide a pharmaceutical composition which comprises a biologically active ingredient-biotin conjugate of the present invention encapsulated with a GRAS encapsulating ingredient. The excipient disclosed in the present invention is pH sensitive and protects the biologically active ingredients, when present, from the harsh acidic milieu of the stomach containing various proteolytic enzymes. Once into the duodenum and into an increased pH environment, the encapsulation falls apart releasing the biologically active ingredient to interact with its receptor. On the other hand, biotin conjugated to the biologically active ingredient interacts with biotin receptor present on the surface of enterocytes.

A further preferred objective of the present invention is to provide a pharmaceutical composition whose transport across the intestinal barrier and bioactivity is independent of particle size. The formulation releases the biologically active ingredient in the alkaline pH of the intestine making it accessible to its receptors.

A further objective of the present invention is process for preparing of a polymer biologically active ingredient complex.

### BRIEF DESCRIPTION OF THE DRAWINGS

The present invention and its various embodiments will now be described with the help of the accompanying drawings.
FIG. 1 illustrates an in vitro assay for enterocyte cell permeability for predicting in vivo absorption of the targeted drugs across the gut.
FIG. 2 represents a graph illustrating the movement of biotin-insulin formulation across polarized and differentiated Caco2 monolayer.
FIG. 3A represents a graph illustrating the internalization of Biotin-Q dots into CHO cells.
FIG. 3B represents a graph illustrating the internalization of Biotin-Q dots into Caco2 cells.
FIG. 4 represents a diagram representing the presence and location of insulin and biotin receptors in various regions of the gut.
FIG. 5 represents a graph illustrating the hypoglycemic effect of therapeutic proteins in a diabetic rat.
FIG. 6 represents a bar diagram describing the distribution of insulin in major organs of a diabetic rat when biotin-insulin formulation is administered orally.
FIG. 7 represents a graph illustrating the movement of gp120-DT fusion protein across the intestinal barrier into systemic circulation.

### DETAILED DESCRIPTION OF THE INVENTION

The detailed description of the present invention is not limited in its application merely to the exemplifications of construction and the arrangement of components set forth in the following description or illustrated in the drawings. The present disclosure is capable of encompassing other embodiments and of being practiced or of being carried out in various ways. Also, the phraseology and terminology used herein is for the purpose of description and should not be regarded as limiting.

The use of the expressions such as "including", "comprising" or "having" and, variations thereof will be understood to encompass the items listed thereafter and equivalents thereof as well as additional items as applicable. The terms such as "a" and "an" herein do not denote a limitation of quantity, but rather denote the presence of at least one of the referenced items. Further, the use of terms "first", "second", and "third", and the like, herein do not denote any order, quantity, or importance, but rather are used to distinguish one element from another.

According to a non limiting-embodiment of the present invention, a pharmaceutical formulation and a process for oral delivery of biologically active ingredients which may include but are not limited to proteins and peptides like insulin or its analogues via biotin receptors present in the gastrointestinal tract of the vertebrate host is disclosed.

According to a non limiting preferred embodiment of the present invention the peptide-biotin complex reacts with each other through a cross-linking agent and a spacer compound so that the biotin does not interfere with the peptides binding to its own receptor.

In accordance with a non limiting preferred aspect of the present invention, the peptide-biotin complex is encapsulated by a encapsulating GRAS ingredient which is stable in the harsh environment of the gastrointestinal tract. The capsule opens up in the alkaline pH of the gut thereby releasing peptide-biotin conjugate, in which the peptide or biotin can bind to its intestinal receptor of the vertebrate host.

According to a non limiting preferred embodiment of the present invention, the peptide-biotin conjugate is transported by carrier-mediated transcyotsis across the intestinal barrier using peptide and biotin receptors. Thus there are two potential receptor systems that partake in absorption of peptide thereby increasing the total bioavailability of peptide in the systemic circulation.

In accordance with a non limiting further preferred aspect of the present disclosure, the peptide-biotin conjugate is preferentially taken up by the lymphatic system for example in case of insulin or its analogues, resulting in a sustained release in systemic circulation and a prolonged therapeutic effect. The uptake of peptide-biotin conjugate by the lymphatic system in the intestinal space which avoids first pass metabolism of liver and results in increasing total bioavailability of the peptide in the systemic circulation.

The complexes of the present invention have the general formula

[{(B-S)ₙ-(A)}ₘ]E

wherein, B is a ligand selected from biotin or an analogue of biotin, which binds to the natural intestinal biotin binding protein or biotin receptor, S is the spacer compound that forms a chelated complex of B to the biologically active ingredient A. In the above described complex n is the molar ratio of biotin or analogues of biotin that are covalently bound to one mol of biologically active ingredient A via a spacer S. The number n varies and is unique for each protein depending upon the reactive amino acid residue available and varies from 1 to 10 molar. On the other hand, m depicts the number of biologically active ingredient spacer complexes encapsulated by the encapsulating GRAS ingredient E. m varies and is unique for each ingredient complex ranging from 1 to 1000.

A process for the production of the complex comprising the following steps:
(a) reacting biotin or its analogue (B) with spacer (S) in equimolar ratio, by stirring for 2 to 4hrs at room temperature thereby forming a biotin spacer complex (B-S) and subsequent removal of non- reacted spacer by overnight dialysis at 2 to 8 °C.
(b) Reacting the resultant biotin spacer complex (B-S) by stirring overnight at 2 to 8 °C with a biologically active ingredient (A) in 1 to 10M ratio thereby forming a (B-S)n-(A) product,
(c) Removing the un-reacted biotin spacer complex (B-S) by dialysis against physiological buffers (pH 6-9) overnight at 2 to 8 °C,
(d) Encapsulating the said product (B-S)ₙ-(A) by gentle stirring overnight at 2 to 8 °C with encapsulating GRAS ingredient (E) in a ratio of 1:5 to 1:40, pH 1 to 10 with pH 4.5 being the preferred pH, to form the complex [{(B-S)ₙ-(A)}ₘ]E.

In another non-limiting exemplified embodiment of the current invention, the pharmaceutical formulation displays the versatility of delivering a range of biologically active ingredients through the peroral route and has the utility to be used as a "kit" for delivering a range of biologically active ingredients, which are hitherto delivered by injections. This potential of the pharmaceutical formulation is not limited by the size or charge of the biologically active ingredient and presence or absence of the receptors in the gastrointestinal tract. This versatility of the invention is exemplified by delivering a 6kDa polypeptide (Insulin) and a >68kDa protein (gp120-DT fusion protein). In one of the preferred embodiment of the invention, in the case of proteins, where receptors for its transcytosis across the GI barrier are present, the described invention (kit) prevents its enzymatic degradation and the conjugated biotin or biotin analogues work in tandem with the protein receptors for its increased movement across the GI barrier into systemic circulation. In yet another embodiment of the invention, where there are no naturally occurring receptors for the protein to be delivered orally, biotin or its analogues when conjugated to the protein, act as carriers and take the cargo (such as protein) across the GI barrier utilizing the transcytotic receptors for biotin or its analogues.

### DETAILED PROCESS FOR MANUFACTURING OF THE FORMULATION AND PROOF OF CONCEPT STUDIES

### Preparation of NHS-biotin

Biotin (5g) was dissolved in 100 ml dry DMSO, plus 2.5 ml triethylamine.

N-hydroxysuccinimide (2.6 gm) was added as a powder to the biotin and reacted overnight with 4.7 gm dicyclohexylcarbodiimide at room temperature. The dicyclohexylurea was removed by filtration. The DMSO was concentrated under reduced pressure and heating, and NHS-biotin precipitated by adding multiple volumes of diethylether. The product, was washed several times with anhydrous ether, dried under vacuum and stored as a white powder.

### Biotin Derivatisation of Peptides

Biotin (90 mg) was dissolved in DMSO (5.0 ml). DIEA (75 uL) was added, followed by TSTU ((O-(N-Succinimidyl)-N,N,N',N'-bis(tetramethylene)uronium hexafluorophosphate) (180 mg). The biotin was activated for 10 min, then 100 mg of the peptide to be conjugated was dissolved in DMSO (2 ml) was added to the activated biotin solution and reacted overnight. The product was purified by repeated dialysis against phosphate buffered saline (PBS) (pH 8.0) to ensure removal of un-reacted biotin. The product was lyophilized and stored under frozen conditions.

### Biotin Derivatisation of Proteins

Biotin (90 mg) was dissolved in DMSO (5.0 ml). DIEA (75 uL) was added, followed by TSTU ((O-(N-Succinimidyl)-N,N,N',N'-bis(tetramethylene)uronium hexafluorophosphate) (180 mg). The biotin was activated for 10 min, then 500 mg of the protein to be conjugated was dissolved in 10 ml PBS and added to the activated biotin solution and reacted overnight. The product was dialysed extensively to ensure removal of un-reacted biotin. The product was lyophilized and stored under frozen conditions.

### Biotin Derivatisation of Insulin

Insulin (300mg) (60 mg/ml) is dissolved in DMSO and DIEA added to 30 ul/ml. NHS-biotin is dissolved at 70 mg/ml in dry DMSO and 0.5 ml immediately added to the insulin solution. The reaction is allowed to proceed for 2 hours after which the DMF is precipitated by addition of 15 ml ethyl acetate. The resultant product is isolated by low speed centrifugation and re-suspended at 50 mg/ml in 0.1 N HCl. Unconjugated biotin is removed by extensive dialysis against PBS. The product was lyophilized and stored under frozen conditions.

### Preparation of biotinyl-gp120-DT

gp120-DT (200 mg) was dissolved at 50 mg/ml in phosphate buffered saline (4 ml). Freshly prepared NHS biotin (20mg) was dissolved 1 ml dry DMSO and immediately added to the gp120-DT. The solution was stirred rapidly for 4 hours at room temperature. The product, biotinyl-gp120, was separated from free biotin by dialysis against phosphate buffered saline. The biotinyl-gp120-diphtheria toxin conjugate was lyophilized and stored dry until used.

### Process for the preparation of polyanionic structures with carboxymethyl dextran and biotin complexes

Carboxy methyl dextran (CMD) was dissolved at 80 mg/ml in distilled water. The mixture was thoroughly mixed by vortexing and the pH titrated to 1-10 with 1 M NaOH, with pH 4.5 being the optimal. Protein-biotin or protein-biotin analogue complex is slowly added to the CMD solution in a ratio ranging from 1:5 - 1:40 and stirred for a further hour. The solution used immediately for the animal experiments or lyophilized and stored dry until used.

### In vivo studies on glucose reduction with orally administered biotinyl-insulin

Wistar rats (190-210 g) were housed at a room temperature of 22±2 °C with 12 h light/dark cycle and 45-50% relative humidity. The animals had ad libitum access to a standard chow diet and water except, wherever indicated. After randomization into various groups, the rats are acclimatized for a period of 2 days in the new environment, before initiation of the experiment.

### Induction of diabetes

Streptozotocin is dissolved at 45 - 55 mg/ml in 0.1 M citrate buffer pH 4.0 and sonicated to make sure of even dissolution. To induce diabetes, freshly prepared Streptozotocin solution is administrated intraperitoneally to the rats (∼200gm) at a dose range of 45 - 55 mg/kg.

### Monitoring plasma glucose and insulin

Blood is withdrawn either from the orbital sinus of conscious rats or from the tail vein of the rat using a 23 gauge needle shaft and subjected to plasma separation. Plasma glucose is measured immediately by colorimetric method. Animals showing blood glucose levels between 250 - 400 mg/dL, are regarded as diabetic, and can be used for subsequent study. Plasma insulin levels are monitored using iso Insulin ELISA kits. At the end of the study, major organs harvested and homogenized in PBS. Clarified supernatants normalized by protein estimation and insulin levels monitored by iso Insulin ELISA assays.

### Visualization of biotin-mediated uptake in the intestine of Wistar rats

The presence of the intestinal biotin receptor-mediated uptake was demonstrated using a gastro-intestinal loop model. Intestinal loops were prepared in anaesthetized Wistar rats and fluorescently labelled compounds instilled into the loops and at various times, the rats were euthanized and the loops removed and prepared for cryostat sectioning. Images captured using confocal microscopy.

### Oral delivery of biotinyl-gp120-DT

The effectiveness of biotin as an oral delivery agent was tested by administering biotinyl-gp120-diphtheria toxin conjugate to non-immunized control rats. Briefly biotinyl-gp120-diphtheria toxin conjugate in the range of 1 - 2mg, encapsulated with CM Dextran pH 4.5 in the ratio of 1:5 - 1:40 was orally administered to Wistar rats. As a positive control, conjugated protein (100ug) in carrier (PBS) administered intra venously to another set of non immunized rats. The presence of conjugated protein in the systemic circulation was monitored by ELISA analysis of blood taken from the rats over a period of 24h.

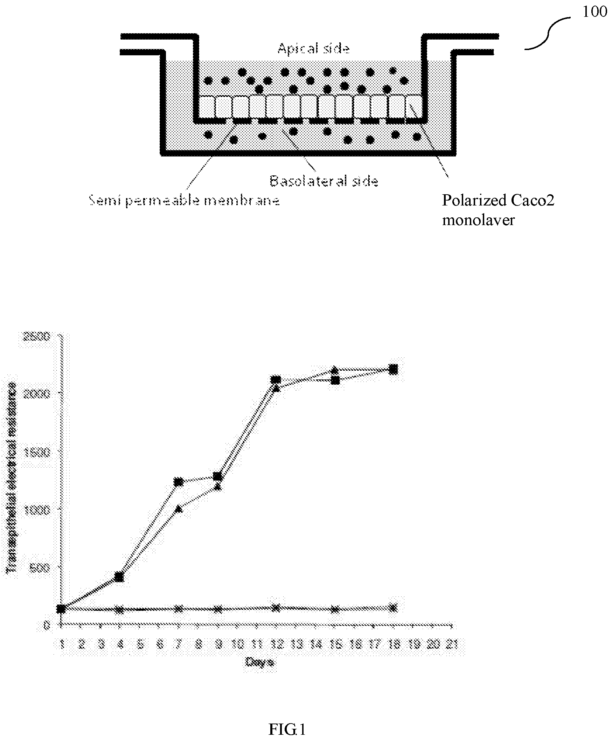

FIG. 1 represents an in vitro enterocyte cell permeability assay (100) for predicting in vivo absorption of the targeted drugs across the gut. According to a non limiting exemplary embodiment of the present invention, colon adenocarcinoma Caco2 cells are cultured on the semi permeable membranes for -18 days where they differentiated to form a Caco2 monolayer which is morphologically and biochemically similar to the intestinal enterocyte monolayer having distinct apical and basolateral membranes. The transepithelial electrical resistance (TEER) between apical and basolateral chambers indicates the tightness of the cells and reaches a maxima in around 15 days preventing any paracellular movement of the drug in between the cells. The targeted drug is added on the apical chamber and its appearance in the basolateral chamber indicates its potential to cross the intestinal barrier.

Typical biologically active ingredients for delivery according to the present disclosure include but not limited to hormones and bioactive peptides such as vasopressin, oxytocin, insulin, testosterone, interferons, somatotrophin, somatostatin, erythropoietin, Colony Stimulating Factors (GCSF, GM-CSF, CSF), PMSG, HCG, inhibin, PAI-1, PAI-2, therapeutic agents such as neomycin, salbutamol, pyrimethamine, penicillin G, methicillin, carbenicillin, pethidine, zylazine, ketamine HCl, GABA, iron dextran, nucleotide analogues or ribozyme.

Biologically active ingredient includes polypeptide such as, insulin, somatostatin, somatostatin derivatives, growth hormones, prolactin, adrenocorticotropic hormone (ACTH), melanocyte stimulating hormone (MSH), thyroid hormone releasing hormone(TRH), thyroid stimulating hormone (TS), luteinizing hormone (LH), follical stimulating hormone (FS), vasopressin, vasopressin derivatives, oxytocin, calcitonin, parathyroid hormone, glucagon, gastrin, secretin, pancrozymin, cholecystokinin, angiotensin, human placental lactogen, human chorionic gonadotropin (HCG), enkephalin, enkaphalin derivatives, endorphin, kyotorphin, interferon γ), interleukins (I, II and III), tufstin, thymopoietin, thymosin, thymostimulin, thymic humoral factor (TFH), serum thymic factor (FTS), and its derivatives, tumor necrosis factor (TNF), colony stimulating factor (CSF), motilin, dinorphim, bombesin, neurotensin, cerulein, bradykinin, urokinase, asparaginase, kallikrein, substance P and its antagonists, nerve growth factor, blood coagulation factors VIII and IX, lysozyme chloride, polymyxin B, colistin, gramicidin, bacitracin, protein synthesis stimulating peptides, gastric inhibitory polypeptide (GIP), vasoactive intestinal polypeptide (VIP), platelet-derived growth (PDGF), growth hormone, growth hormone factor (GRF, somatocrinin), bone morphogenic factor (BMP), epidermal growth factor (EGF), Fab and Fc portions of mAb's etc.

Accordingly, the present invention provides a new set of biologically active ingredients to which a biotin molecule, or analogue thereof, has been conjugated. These biotin conjugates are suitable for oral delivery to a vertebrate host whereby as they can utilize the aforementioned biotin receptor system for uptake binding and uptake of the pharmaceutical from the intestine into the circulation of the vertebrate host.

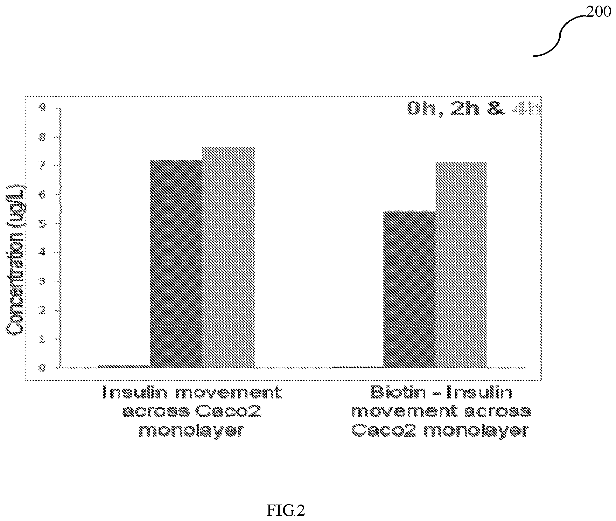

FIG. 2 represents a graph (200) describing the movement of insulin and biotin-insulin across differentiated Caco2 monolayer. In accordance with a non limiting exemplary aspect of the present invention, the biologically active ingredient dissolved in buffer solution are added on the apical chamber and the amount of biologically active ingredient appearing in the basolateral chamber is monitored by Enzyme-linked immunosorbent assay (ELISA). The amount of biotin-insulin formulation appearance in the basolateral chamber increased from 0 to 4 hours indicates the potential of biotin-insulin formulation to bind to biotin receptors on the Caco2 cells and the movement of the biotin-insulin formulation across the monolayer. This indicates that binding capacity of biotin to the insulin has the potential to take it across the intestinal barrier. Here, since unmodified insulin also appears to cross the monolayer, it is difficult to infer whether movement is through biotin receptors only.

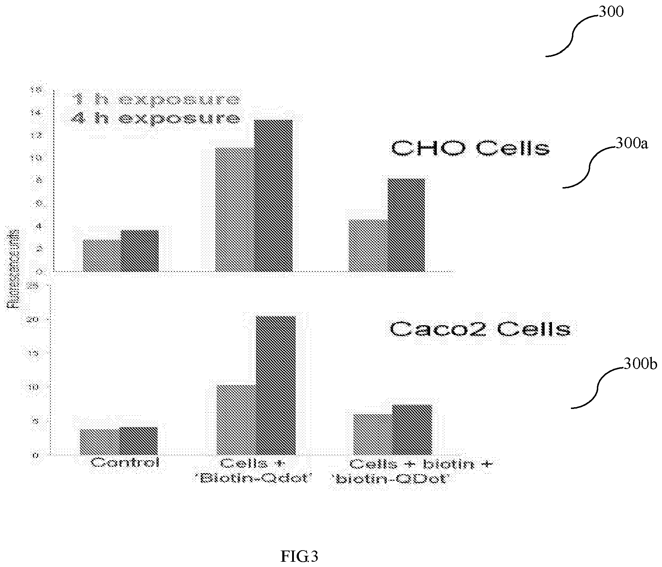

FIG. 3a represents a graph (300a) illustrating the internalization of Biotin-Q dots into CHO cells. Biotin Q dots represent biotin conjugated to a drug moiety and since the size of the Q dots is similar to a small drug, internalization of these particles into the cells cannot be via SMVT. The only way Biotin Q dots can cross the barrier is through carrier mediated endocytosis utilizing biotin receptors. Internalization of Biotin-Q dots into these cells indicates the presence of the biotin receptor and the ability of the biotin to take a drug sized molecule into the cells. These cells represent 1 hour and 4 hour internalization of Biotin-Q dots, in which the internalization of Biotin-Q dots is increased with the increased time interval. When non conjugated biotin is added to the cells before Biotin-Q dots, internalization is inhibited indicating that biotin mediated internalization of Q-dots is specifically using biotin receptors and not through any other pathway. Similarly, FIG. 3b is a graph illustrating the internalization of Biotin-Q dots into Caco₂ cells. These cells are intestinal cell lines and internalization of Biotin-Q dots into these cells indicate the presence of the biotin receptor and the ability of the biotin to take a drug sized molecule into the cells.

Suitable analogues of biotin may include but not limited to biotin, iminobiotin, biocytin hydrazide, biotin hydrazide, biocytin, 5-(Biotinamido)pentylamine, Sulfo-NHS(n-Hydroxysuccinimidyl)-Biotin, Sulfo-HNS-hexanyl-biotin (Sulfo-NHS-LD-Biotin), NHS-Biotin, Pentafluorophenyl-biotin, Pentafluorophenyl-polyethylenoxide-biotin, NHS -biotin Trifluoroacetamide, NHS-Iminobiotin trifluoroacetamide, maleimido-polyethylenoxide biotin, maleimido-polyethylenoxide iminobiotin, chloroacetylated biotin, phosphonoacetatyl-1'N-biotin, biocytin

Suitable extended spacers for the conjugation of the biologically active ingredient to biotin may include but not limited to disuccinimidyl suberate (DSS), bis(sulfosuccinimidyl) suberate (BSS), ethylene glycolbis(succinimidylsuccinate) (EGS), ethylene glycolbis(sulfosuccinimidylsuccinate) (Sulfo-EGS), p-amino-phenylacetic acid, dithiobis(succinimidylpropionate) (DSP), 3,3'-dithiobis(sulfosuccinimidylpropionate) (DTSSP), disuccinimidyl tartarate (DST), disulfosuccinimidyl tartarate (Sulfo-DST), bis[2-(succinimidyloxycarbonyloxy)-ethylene] sulfone (BSOCOES), bis[2-(sulfosuccinimidooxycarbonyloxy)-ethylene]sulfone (Sulfo-BSOCOES), dimethyl adipimidate.2 HCl (DMA), dimethyl pimelimidate.2 HCl (DMP), dimethyl suberimidate.2 HCl (DMS), N-succinimidyl(4-iodoacetyl)aminobenzoate (SIAB), succinimidyl 4-(p-maleimidophyl)butyrate (SMPB).

These biotin -drug conjugates are suitable for oral delivery to a vertebrate host whereby as they can utilize the aforementioned biotin receptor system for uptake binding and uptake of the pharmaceutical from the intestine into the circulation of the vertebrate host.

Such encapsulating GRAS ingredients include but are not limited to carboxymethyl dextran, carrageenan, inulin, carboxymethy cellulose, starch and derivatives thereof; chondroitin sulfate, styrene-maleic anhydride copolymer, divinylether-maleic anhydride copolymer, poly-glutamic acid, polyaspartic acid, polylactic acid.

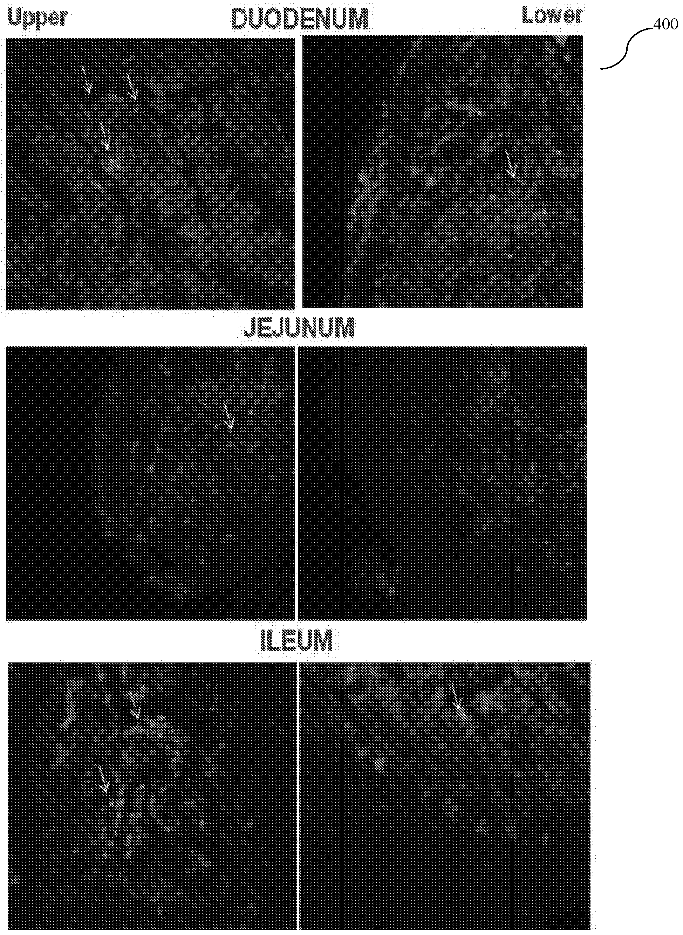

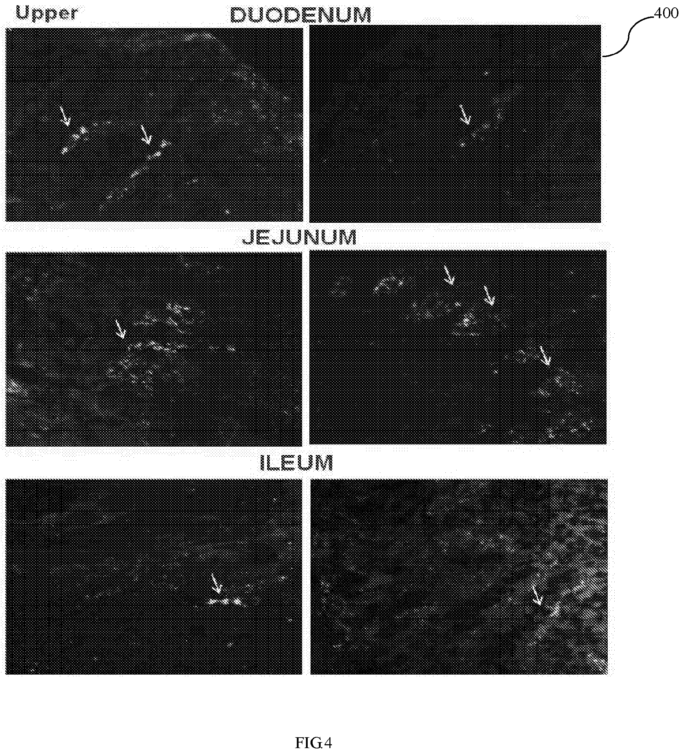

FIG. 4 is a diagram representing the presence and location of insulin (red) and biotin (green) receptors in various regions of the gut (400). As is clear from the images insulin receptors are located in the duodenum and ileum whereas biotin receptors are present all along the length of the gastrointestinal tract including jejunum. According to the present disclosure, insulin formulation is transported across the intestinal barrier using a combination of both the receptors which increases the length of the absorptive surface. Further, it is these receptors which are responsible for the movement of the biotin-insulin formulation across the Caco2 cells described in Fig 2.

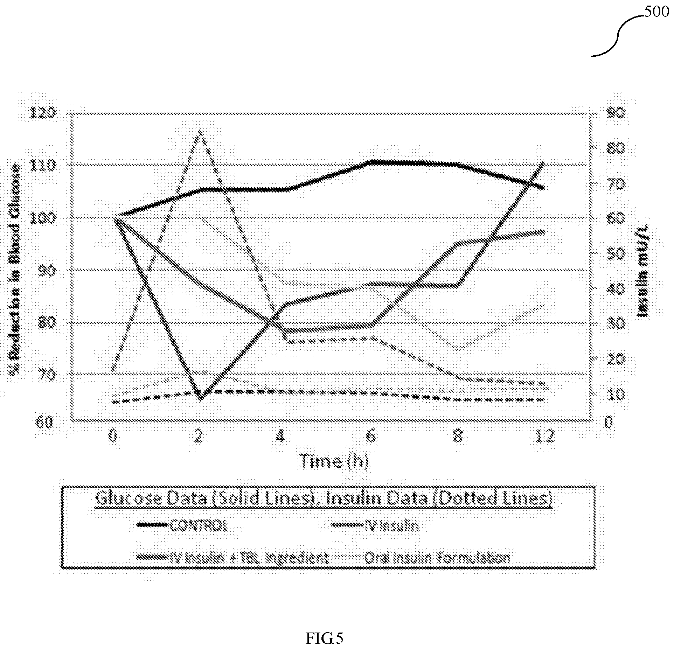

FIG. 5 represents a graph (500) illustrating the hypoglycemic effect of oral insulin formulation in a diabetic rat study. Here, as controls, set of diabetic rats were injected with insulin or insulin conjugated with biotin, represented by solid dark grey colored lines showing a sharp and short lived reduction in blood glucose levels. Hypoglycemic effect in insulin conjugated with biotin indicates that biotin conjugation did not alter its hypoglycemic activity. However, the reduction in blood glucose was not as rapid as unmodified insulin and the effect lasted up to 4 hours after administration. In another set, diabetic rats were orally administered with a biotin-insulin formulation represented by a solid light grey colored line. Oral administration of insulin formulation resulted in sustained and prolonged hypoglycemic effect for up to 8 hours. An explanation for this sustained activity could be the utilization of both insulin and biotin receptors which are present throughout the gut thereby increasing the absorptive length for the formulation. The levels of plasma insulin when biotin insulin was injected is represented by dotted dark grey line and when biotin insulin formulation was orally administered represented by a dotted light grey colored line.

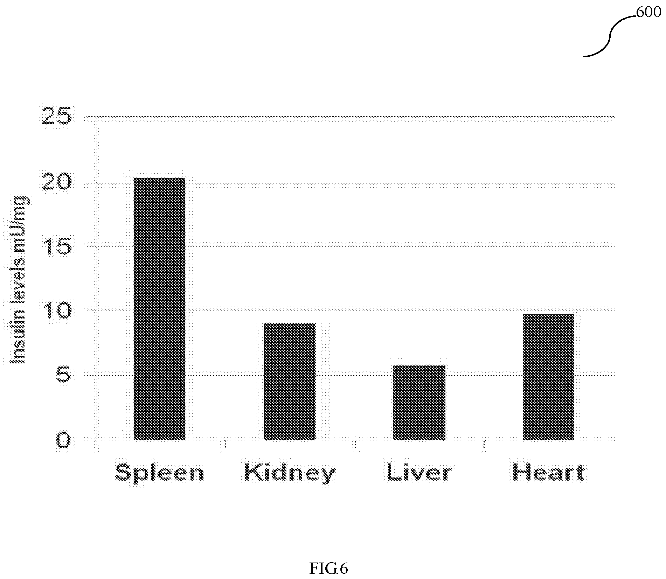

FIG. 6 reflects a bar diagram (600) describing the distribution of insulin in major organs of diabetic rats when biotin-insulin formulation was administered orally. These include spleen, kidney, liver and heart. The levels of insulin are observed to be highest in the spleen while compared to liver and the other organs. This observation indicates that the insulin after crossing the intestinal barrier is taken up preferentially by the lymphatic circulation. This also explains the lower serum levels of insulin in diabetic rats that were orally administered with biotin insulin formulations.

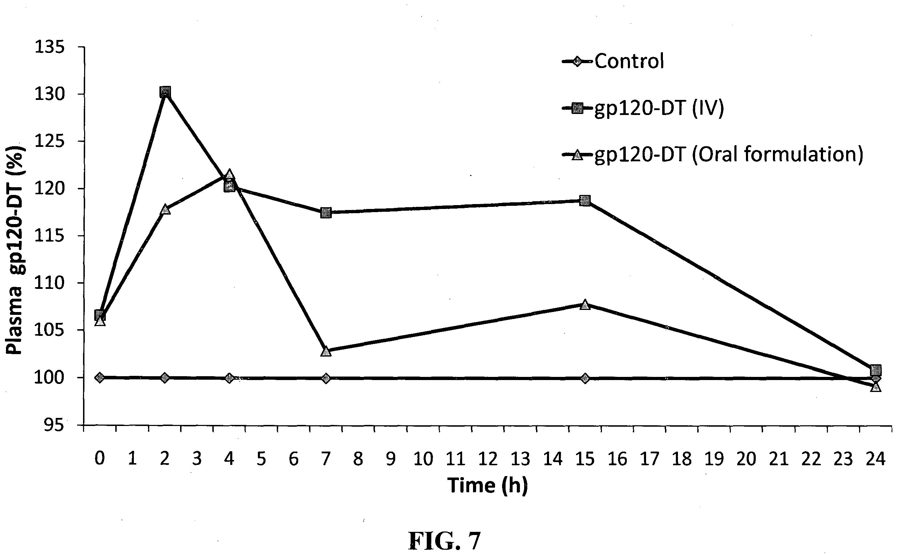

FIG.7, represents a graph (700) indicating the movement of biotinyl gp120.diptheria toxin fusion protein (>68kDa) from across the intestinal barrier into systemic circulation. Fusion protein after conjugation to biotin and encapsulation with the GRAS encapsulating ingredient, was administered orally to non immunized Wistar rats. As a positive control, biotinyl fusion protein was also administered intra venously in one set of animals. Over a course of 24h, blood samples were collected and levels of biotinyl fusion protein determined using ELISA analysis. Appearance of fusion protein in blood plasma after oral administration indicated the capability of the formulation to facilitate the movement of a >68kDa protein across the intestinal barrier into systemic circulation.

### REFERENCES:

Russell-Jones, G. J., Westwood, S. W. & Habberfield, A. 1995. Vitamin B12 mediated oral delivery systems for Granulocyte Colony Stimulating Factor and Erythropoietin. Bioconjugate Chemistry, 6, 459-465
Russell-Jones, G. J. (1996) Utilisation of the Natural Mechanism for Vitamin B12 uptake for the oral delivery of therapeutics. Eur. J. Pharm. Biopharm. 42, 241-249.
Chalasani, K. B., Russell-Jones, G. J., Jain, A., K., Diwan, P. V., and Jain, S. K. 2007 Effective oral delivery of insulin in animal models using vitamin B12-coated dextran nanoparticles. J. Control. Rel. 2007 122, 141-50
Said, Hamid M., Alvaro Ortiz, Eric McCloud, David Dyer, Mary Pat Moyer, and Stanley Rubin. 2008. Biotin uptake by the human colonic epithelial NCM460: a carrier-mediated process shared with pantothenic acid. Am J Physiol. 275: C1365-71
USP 2006/0127310 Amplification of biotin-mediated targeting. Russell-Jones, G. and Mcewan,J. Access Pharmaceuticals US.
USP 2005/0220757 Vitamin directed targeting therapy. Russell-Jones, G and Mcewan, J. Biotech Australia Pty. Ltd.

## Claims

1. A complex of general formula:
[{(B-S)ₙ-(A)}ₘ]E
wherein, B is a ligand selected from biotin or an analogue of biotin, which binds to the natural intestinal biotin binding protein or biotin receptor,
A is a biologically active ingredient,
S is a spacer compound that covalently binds B to the biologically active ingredient A,
E is an encapsulating pH sensitive excipient that protects the biologically active ingredient A from the harsh acidic milieu of the stomach containing various proteolytic enzymes,
n is the molar ratio of biotin or analogues of biotin, and
m is the number of biologically active ingredient-spacer-biotin complexes encapsulated by the encapsulating pH sensitive excipient E.

2. The complex as claimed in claim 1, wherein said biologically active ingredient for delivery includes hormones and bioactive peptides.

3. The complex as claimed in claim 2, wherein said hormones and bioactive peptides comprise vasopressin, oxytocin, insulin, testosterone, interferons, somatotrophin, somatostatin, erythropoietin, Colony Stimulating Factors (GCSF, GM-CSF, CSF), PMSG, HCG, inhibin, PAI-1, PAI-2, therapeutic agents such as neomycin, salbutamol, pyrimethamine, penicillin G, methicillin, carbenicillin, pethidine, zylazine, ketamine HCl, GABA, iron dextran, nucleotide analogues or ribozyme.

4. The complex as claimed in claim 1, wherein suitable analogues of biotin (B) includes biotin, iminobiotin, biocytin hydrazide, biotin hydrazide, biocytin, 5-(Biotinamido)pentylamine, Sulfo-NHS(n-Hydroxysuccinimidyl)-Biotin, Sulfo-HNS-hexanyl-biotin (Sulfo-NHS-LD-Biotin), NHS-Biotin, Pentafluorophenyl-biotin, Pentafluorophenyl-polyethylenoxide-biotin, NHS-biotin Trifluoroacetamide, NHS-Iminobiotin trifluoroacetamide, maleimido-polyethylenoxide biotin, maleimido-polyethylenoxide iminobiotin, chloroacetylated biotin, phosphonoacetatyl-1 'N-biotin, biocytin.

5. The complex as claimed in claim 1, wherein said spacer (S) for the conjugation of the biologically active ingredient to biotin is selected from disuccinimidyl suberate (DSS), bis(sulfosuccinimidyl) suberate (BSS), ethylene glycolbis(succinimidylsuccinate) (EGS), ethylene glycolbis(sulfosuccinimidylsuccinate) (Sulfo-EGS), p-amino-phenylacetic acid, dithiobis(succinimidylpropionate) (DSP), 3,3'dithiobis(sulfosuccinimidylpropionate) (DTSSP), disuccinimidyl tartarate (DST), disulfosuccinimidyl tartarate (Sulfo-DST), bis[2-(succinimidyloxycarbonyloxy)-ethylene]sulfone (BSOCOES), bis[2-(sulfosuccinimidooxycarbonyloxy)-ethylene]sulfone (Sulfo-BSOCOES), dimethyl adipimidate.2 HCl (DMA), dimethyl pimelimidate.2 HCl (DMP), dimethyl suberimidate.2 HCl (DMS), N-succinimidyl(4-iodoacetyl)aminobenzoate (SIAB), succinimidyl 4-(p-maleimidophyl)butyrate (SMPB).

6. The complex as claimed in claim 1, wherein the said encapsulating pH sensitive excipient includes carboxymethyl dextran, carrageenan, inulin, carboxymethyl cellulose, starch and derivatives thereof; chondroitin sulfate, styrene-maleic anhydride copolymer, divinylether-maleic anhydride copolymer, poly-glutamic acid, polyaspartic acid, polylactic acid.

7. The complex as claimed in claim 1, wherein one mole of biologically active ingredient A is covalently bound to one mole of a spacer S.

8. The complex as claimed in claim 1, wherein the number n varies and is unique for each protein depending upon the reactive amino acid residues available.

9. The complex as claimed in claim 8, wherein n varies from 1 to 10 molar.

10. The complex as claimed in claim 1, wherein m varies and is unique for each biologically active ingredient complex.

11. The complex as claimed in claim 10, wherein m ranges from 1 to 1000.

12. A process for the production of the complex as claimed in claim 1, comprising the following steps:
(a) reacting biotin or its analogue (B) with spacer (S) in equimolar ratio, by stirring for 2 to 4h at room temperature thereby forming a biotin spacer complex (B-S) and subsequent removal of non-reacted spacer by overnight dialysis at 2 to 8°C.
(b) Reacting the resultant biotin spacer complex (B-S) by stirring overnight at 2 to 8 °C with a biologically active ingredient (A) in 1 - 10M ratio thereby forming a (B-S)n-(A) product,
(c) Removing the un-reacted biotin spacer complex (B-S) by dialysis against physiological buffers (pH 6-9) overnight at 2 to 8°C,
(d) Encapsulating the said product (B-S)ₙ-(A) by gentle stirring overnight at 2 to 8 °C with encapsulating pH sensitive excipient (E) in a ratio of 1:5 to 1:40, pH 1-10 with 4.5 being the preferred pH, to form the complex [{(B-S)ₙ-(A)}ₘ]E.

## Patentansprüche

1. Komplex der allgemeinen Formel:
[{(BS)ₙ- (A)}ₘ]E,
wobei B ein Ligand ist, ausgewählt aus Biotin oder einem Analogon von Biotin, das an das natürliche intestinale Biotin-bindende Protein oder den Biotin-Rezeptor bindet,
A ein biologischer Wirkstoff ist,
S eine Spacer-Verbindung ist, die B kovalent an den biologischen Wirkstoff A bindet,
E ein verkapselnder pH-empfindlicher Hilfsstoff ist, der den biologischen Wirkstoff A vor dem stark sauren Milieu des Magens, enthaltend verschiedene proteolytische Enzyme, schützt,
n das Molverhältnis von Biotin oder Analoga von Biotin ist und
m die Anzahl der biologischen Wirkstoff-Spacer-Biotin-Komplexe ist, die durch den verkapselnden pH-empfindlichen Hilfsstoff E verkapselt werden.

2. Komplex nach Anspruch 1, wobei der biologische Wirkstoff zur Abgabe Hormone und bioaktive Peptide einschließt.

3. Komplex nach Anspruch 2, wobei die Hormone und bioaktiven Peptide Vasopressin, Oxytocin, Insulin, Testosteron, Interferone, Somatotropin, Somatostatin, Erythropoietin, koloniestimulierende Faktoren (GCSF, GM-CSF, CSF), PMSG, HCG, Inhibin, PAI-1, PAI-2, Therapeutika wie Neomycin, Salbutamol, Pyrimethamin, Penicillin G, Methicillin, Carbenicillin, Pethidin, Zylazin, Ketamin HCl, GABA, Eisendextran, Nukleotidanaloga oder Ribozym umfassen.

4. Komplex nach Anspruch 1, wobei geeignete Analoga von Biotin (B) Biotin, Iminobiotin, Biocytinhydrazid, Biotinhydrazid, Biocytin, 5-(Biotinamido)pentylamin, Sulfo-NHS(n-Hydroxysuccinimidyl)-Biotin, Sulfo-HNS-Hexanylbiotin (Sulfo-NHS-LD-Biotin), NHS-Biotin, Pentafluorphenylbiotin, Pentafluorphenylpolyethylenoxidbiotin, NHS-Biotintrifluoracetamid, NHS-Iminobiotintrifluoracetamid, Maleimidpolyethylenoxidbiotin, Maleimidpolyethylenoxidiminobiotin, chloracetyliertes Biotin, Phosphonoacetatyl-1 'N-Biotin, Biocytin umfassen.

5. Komplex nach Anspruch 1, wobei der Spacer (S) für die Konjugation des biologischen Wirkstoffs an Biotin ausgewählt ist aus Disuccinimidylsuberat (DSS), Bis(sulfosuccinimidyl)suberat (BSS), Ethylenglycolbis(succinimidylsuccinat) (EGS), Ethylenglycolbis(sulfosuccinimidylsuccinat) (Sulfo-EGS), p-Aminophenylessigsäure, Dithiobis(succinimidylpropionat) (DSP), 3,3'-Dithiobis(sulfosuccinimidylpropionat) (DTSSP), Disuccinimidyltartrat (DST), Disulfosuccinimidyltartarat (Sulfo-DST), Bis[2-(succinimidyloxycarbonyloxy)-ethylen]sulfon (BSOCOES), Bis[2-(sulfosuccinimidooxycarbonyloxy)-ethylen]sulfon (Sulfo-BSOCOES), Dimethyladipimidat.2 HCl (DMA), Dimethylpimelimidat.2 HCl (DMP), Dimethylsuberimidat.2 HCl (DMS), N-Succinimidyl(4-iodacetyl)aminobenzoat (SIAB), Succinimidyl-4-(p-maleimidophyl)butyrat (SMPB).

6. Komplex nach Anspruch 1, wobei der verkapselnde pH-empfindliche Hilfsstoff Carboxymethyldextran, Carrageenan, Inulin, Carboxymethylcellulose, Stärke und Derivate davon; Chondroitinsulfat, Styrolmaleinsäureanhydrid-Copolymer, Divinylether-Maleinsäureanhydrid-Copolymer, Polyglutaminsäure, Polyasparaginsäure, Polymilchsäure einschließt.

7. Komplex nach Anspruch 1, wobei ein mol von biologischem Wirkstoff A kovalent an ein Mol eines Spacer S gebunden ist.

8. Komplex nach Anspruch 1, wobei die Anzahl n variiert und für jedes Protein in Abhängigkeit von den verfügbaren reaktiven Aminosäureresten spezifisch ist.

9. Komplex nach Anspruch 8, wobei n von 1 bis 10 mol variiert.

10. Komplex nach Anspruch 1, wobei m variiert und für jeden Polypeptidkomplex spezifisch ist.

11. Komplex nach Anspruch 10, wobei m im Bereich von 1 bis 1000 ist.

12. Verfahren zur Herstellung des Komplexes nach Anspruch 1, umfassend die folgenden Schritte:
(a) Reagieren von Biotin oder seines Analogons (B) mit Spacer (S) in äquimolarem Verhältnis, wobei 2 bis 4 Stunden lang bei Raumtemperatur gerührt wird, wodurch sich ein Biotin-Spacer-Komplex (B-S) bildet und anschließende Entfernung von nicht reagiertem Spacer durch Dialyse über Nacht bei 2 bis 8°C.
(b) Reagieren des resultierenden Biotin-Spacer-Komplexes (B-S) durch Rühren über Nacht bei 2 bis 8ºC mit einem biologischen Wirkstoff (A) in einem Verhältnis von 1-10 M, wodurch sich ein (B-S)ₙ-(A) Produkt bildet,
(c) Entfernen des nicht reagierten Biotin-Spacer-Komplexes (B-S) durch Dialyse gegen physiologische Puffer (pH 6-9) über Nacht bei 2 bis 8 °C,
(d) Verkapseln des Produkts (B-S)ₙ-(A) durch leichtes Rühren über Nacht bei 2 bis 8°C mit verkapselndem pH-empfindlichen Hilfsstoff (E) in einem Verhältnis von 1:5 bis 1:40, pH 1-10, wobei 4,5 der bevorzugte pH-Wert ist, um den Komplex [{(B-S)ₙ-(A)}ₘ]E zu bilden.

## Revendications

1. Complexe de formule générale :
[{(B-S)ₙ-(A)}ₘ]E
dans laquelle B est un ligand sélectionné parmi la biotine ou un analogue de la biotine, qui se lie à la protéine de liaison de la biotine intestinale naturelle ou au récepteur de la biotine,
A est un ingrédient biologiquement actif,
S est un composé espaceur qui lie de manière covalente B à l'ingrédient biologiquement actif A,
E est un excipient d'encapsulation sensible au pH qui protège l'ingrédient biologiquement actif A du milieu acide hostile de l'estomac contenant diverses enzymes protéolytiques,
n est le rapport molaire de la biotine ou des analogues de la biotine, et
m est le nombre de complexes ingrédient biologiquement actif-espaceur-biotine encapsulés par l'excipient d'encapsulation sensible au pH E.

2. Complexe selon la revendication 1, dans lequel ledit ingrédient biologiquement actif pour l'administration comprend des hormones et des peptides bioactifs.

3. Complexe selon la revendication 2, dans lequel lesdites hormones et peptides bioactifs comprennent la vasopressine, l'ocytocine, l'insuline, la testostérone, les interférons, la somatotrophine, la somatostatine, l'érythropoïétine, les facteurs de stimulation de colonie (GCSF, GM-CSF, CSF), PSMG, HCG, l'inhibine, PAI-1, PAI-2, des agents thérapeutiques tels que la néomycine, le salbutamol, la pyriméthamine, la pénicilline G, méthicilline, la carbénicilline, la péthidine, la zylazine, la kétamine HCl, le GABA, le dextrane de fer, les analogues de nucléotides ou le ribozyme.

4. Complexe selon la revendication 1, dans lequel les analogues appropriés de la biotine (B) comprennent la biotine, l'iminobiotine, l'hydrazide de biocytine, l'hydrazide de biotine, la biocytine, la 5-(biotinamido)-pentylamine, la sulfo-NHS-(n-hydroxysuccinimidyl)-biotine, la sulfo-HNS-hexanyl-biotine (sulfo-NHS-LD-biotine), la NHS-biotine, la pentafluorophényl-biotine, la pentafluoro-phényl-polyéthylènoxide-biotine, le trifluoroacétamide de NHS-biotine, le trifluoroacétamide de NHS-iminobiotine, la maléimido-polyéthylènoxide-biotine, la maléimido-polyéthylènoxide iminobiotine, la biotine chloroacétylée, la phosphonoacétatyl-1'N-biotine, la biocytine.

5. Complexe selon la revendication 1, dans lequel ledit espaceur (S) pour la conjugaison de l'ingrédient biologiquement actif avec la biotine est sélectionné parmi le subérate de disuccinimidyle (DSS), le subérate de bis (sulfosuccinimidyle) (BSS), l'éthylène-glycol-bis(succinate de succinimidyle) (EGS), l'éthylène-glycol-bis(succinate de sulfosuccinimidyle) (Sulfo-EGS), l'acide p-amino-phényl-acétique, le dithiobis(propionate de succinimidyle) (DSP), le 3,3'dithiobis(propionate de sulfosuccinimidyle) (DTSSP), le tartrate de disuccinimidyle (DST), le tartrate de disulfosuccinimidyle (Sulfo-DST), la bis[2-(succinimidyl-oxycarbonyloxy)-éthylène]sulfone (BSOCOES), la bis[2-(sulfo-succinimido-oxycarbonyloxy)-éthylène]sulfone (Sulfo-BSOCOES), l'adipimidate de diméthyle.2 HCl (DMA), le pimélimidate de diméthyle.2 HCl (DMP), le subérimidate de diméthyle.2 HCl (DMS), le (4-iodoacétyl)aminobenzoate de N-succinimidyle (SIAB), le 4-(p-maléimidophyl)butyrate de succinimidyle (SMPB).

6. Complexe selon la revendication 1, dans lequel ledit excipient d'encapsulation sensible au pH comprend du carboxyméthyldextrane, du carraghénane, de l'inuline, de la carboxyméthylcellulose, de l'amidon et ses dérivés, du sulfate de chondroïtine, un copolymère styrène-anhydride maléique, un copolymère divinyléther-anhydride maléique, de l'acide polyglutamique, de l'acide polyaspartique, de l'acide polylactique.

7. Complexe selon la revendication 1, dans lequel une mole d'ingrédient biologiquement actif A est liée de manière covalente à une mole d'un espaceur S.

8. Complexe selon la revendication 1, dans lequel le nombre n varie et est unique pour chaque protéine en fonction des résidus d'acides aminés réactifs disponibles.

9. Complexe selon la revendication 8, dans lequel n varie entre 1 et 10 en molarité.

10. Complexe selon la revendication 1, dans lequel m varie et est unique pour chaque complexe d'ingrédient biologiquement actif.

11. Complexe selon la revendication 10, dans lequel m est compris entre 1 et 1000.

12. Processus de production du complexe tel que défini selon la revendication 1, comprenant les étapes suivantes :
(a) faire réagir de la biotine ou son analogue (B) avec un espaceur (S) dans un rapport équimolaire, en agitant pendant 2 à 4 heures à température ambiante pour ainsi former un complexe biotine-espaceur (B-S) et retirer ensuite l'espaceur n'ayant pas réagi par une dialyse pendant une nuit à une température de 2 à 8°C,
(b) faire réagir le complexe biotine-espaceur (B-S) résultant en agitant pendant une nuit à une température de 2 à 8°C avec un ingrédient biologiquement actif (A) dans un rapport 1 - 10M pour ainsi former un produit (B-S)ₙ-(A),
(c) retirer le complexe biotine-espaceur (B-S) n'ayant pas réagi par une dialyse à l'aide de tampons physiologiques (pH 6 à 9) pendant une nuit à une température de 2 à 8°C,
(d) encapsuler ledit produit (B-S)ₙ-(A) par une agitation douce pendant une nuit à une température de 2 à 8°C avec l'excipient d'encapsulation sensible au pH (E) dans un rapport de 1:5 à 1:40, un pH de 1 à 10 avec 4,5 étant le pH préféré, pour former le complexe [{(B-S)ₙ-(A)}ₘ]E.
